# EUROPÄISCHE PATENTSCHRIFT

(11) **EP 2 331 483 B1**
(45) Veröffentlichungstag und Bekanntmachung des Hinweises auf die Patenterteilung: **02.03.2016**
(21) Anmeldenummer: 09804525.5
(22) Anmeldetag: 07.08.2009
(51) Int. Cl.: C04B 35/64, C04B 35/10, C04B 35/48, A61L 27/10, B24C 1/08, C04B 41/51, C04B 41/88, C23C 4/08, A61L 27/30, A61F 2/30, A61F 2/44, C04B 35/622

(54) **VERFAHREN ZUM HERSTELLEN EINES KERAMIKBAUTEILS**
METHOD FOR PRODUCING A CERAMIC COMPONENT
PROCÉDÉ DE RÉALISATION D'UN ÉLÉMENT CÉRAMIQUE

(30) Priorität: 07.08.2008 WO PCT/EP2008/006524
(43) Veröffentlichungstag der Anmeldung: 15.06.2011
(73) Patentinhaber: Waldemar Link GmbH & Co. KG, 22339 Hamburg (DE)
(72) Erfinder: LINK, Helmut D., 22397 Hamburg (DE)
(74) Vertreter: Glawe, Delfs, Moll
(86) Internationale Anmeldenummer: PCT/EP2009/005746
(87) Internationale Veröffentlichungsnummer: WO 2010/015414

(56) Entgegenhaltungen:
- EP-A- 0 421 084
- EP-A- 0 633 440
- EP-A- 1 440 669
- DE-A- 3 516 411
- DE-A- 4 322 083
- DE-A- 4 322 085
- DE-A- 19 755 536
- US-A1- 2004 246 088

## Beschreibung

Die Erfindung betrifft ein Verfahren zum Herstellen eines Keramikbauteils. Bei dem Verfahren wird ein Keramikausgangsmaterial in Pulverform bereitgestellt und in eine die Gestalt des Keramikbauteils vorgebenden Form eingebracht. Das Keramikbauteil wird vorgesintert, aus der Form herausgelöst und anschließend bei einer Temperatur gesintert, die höher ist als die Temperatur des Vorsinterns.

Ein solches Verfahren zum Herstellen eines Keramikbauteils ist beispielsweise aus EP 0 421 084, das die Grundlage des Oberbegriffs von Anspruch 1 bildet, bekannt. Es zeigt sich, dass auf diese Weise hergestellte Keramikbauteile eine geringe Oberflächenrauigkeit haben und dass es deswegen schwierig ist, eine Beschichtung festhaftend auf das Keramikbauteil aufzubringen.

Der Erfindung liegt die Aufgabe zu Grunde, ein Verfahren zum Herstellen eines Keramikbauteils vorzustellen, bei dem ein erfindungsgemäß hergestelltes Keramikbauteil eine bessere Ausgangsbasis zum Aufbringen einer Beschichtung bietet. Ausgehend vom eingangs genannten Stand der Technik wird die Aufgabe gelöst durch die Merkmale der unabhängigen Ansprüche. Vorteilhafte Ausführungsformen finden sich in den Unteransprüchen. Erfindungsgemäß wird für den Schritt des Vorsinterns eine Temperatur zwischen 880°C und 980°C gewählt. Nach dem Vorsintern und vor dem Sintern wird die Oberfläche des Keramikbauteils mit einem Strahlgut behandelt.

Zur Herstellung von Keramikbauteilen wird zunächst ein pulverförmiges Keramikausgangsmaterial in eine Form gefüllt, die die Gestalt des Keramikbauteils vorgibt. Da sich das Volumen des Keramikbauteils durch nachfolgende Bearbeitungsschritte verringert, ist die Form größer als das fertige Keramikbauteil. Eine stabile innere Struktur des Keramikbauteils wird durch nachfolgendes Sintern erreicht. Durch das Sintern entstehen feste Verbindungen zwischen den Partikeln des Pulvers.

Das Sintern wird in mehreren Schritten durchgeführt. In einem ersten Schritt wird das Keramikbauteil bei einer niedrigeren Temperatur vorgesintert, so dass sich lediglich schmale Brücken zwischen den Körnern des Pulvers herausbilden. Für das erfindungsgemäße Verfahren soll die Stabilität der schmalen Brücken gerade derart sein, dass die Behandlung der Oberfläche mit einem Strahlgut zu einer erhöhten Oberflächenrauigkeit führt. Ist die Stabilität der Brücken zu gering, kann das Keramikbauteil in mehrere Bestandteile zerbersten, ist die Stabilität der Brücken zu hoch, reicht die Behandlung mit dem Strahlgut nicht aus, um die Oberflächenstruktur des Keramikbauteils zu verändern. Versuche haben gezeigt, dass die gewünschte Veränderung der Oberflächenstruktur durch das Strahlgut dann eintritt, wenn für das Vorsintern eine Temperatur zwischen 880°C und 980°C gewählt wird. Die innere Struktur des Keramikbauteils ist dann gerade derart, dass mit dem Strahlgut Bruchstücke der gewünschten Größe aus dem Bauteil herausgebrochen werden können.

Allgemein steigt die Festigkeit der Verbindung zwischen den Partikeln des Pulvermaterials an, wenn die Temperatur des Vorsinterns erhöht wird. Gute Ergebnisse mit dem erfindungsgemäßen Verfahren wurden erzielt, wenn die Temperatur des Vorsinterns größer war als 900°C bzw. kleiner war als 950°C.

Um das Keramikbauteil mit einer Titanlegierung beschichten zu können, sollte die Oberflächenrauigkeit Rₐ größer sein als 2,5 µm (WO 2009/036845). Die Rauigkeitsangaben im Rahmen der Erfindung beziehen sich auf die mittlere Rauigkeit Rₐ gemäß DIN EN ISO 4288 und 3274 und auf das fertige Keramikbauteil nach dem Sintern. Die mit dem erfindungsgemäßen Verfahren erzielte Oberflächenrauigkeit hängt von den bei der Behandlung mit dem Strahlgut gewählten Parametern ab, wie beispielsweise Partikelgröße des Strahlguts und Geschwindigkeit, mit der die Partikel auf das Keramikbauteil auftreffen. Vorzugsweise werden diese Parameter bei dem erfindungsgemäßen Verfahren so eingestellt, dass die Oberflächenrauigkeit Rₐ des fertigen Keramikbauteils größer ist als 2,5 µm. Damit die Beschichtung in einem Arbeitsgang deckend aufgetragen werden kann, sollte die Oberflächenrauigkeit Rₐ des fertigen Keramikbauteils nicht größer sein als 7 µm.

Im Rahmen der Erfindung kann die gesamte Oberfläche des Keramikbauteils mit dem Strahlgut behandelt werden. Dies ist dann sinnvoll, wenn die gesamte Oberfläche des Keramikbauteils mit einer Beschichtung versehen werden soll. Häufig soll aber nur ein Teil der Oberfläche beschichtet werden, während ein anderer Teil der Oberfläche frei von Beschichtungen sein soll. Dies gilt beispielsweise für Endoprothesenkomponenten, deren Oberfläche zum Teil eine Verbindung mit Knochenmaterial eingehen soll, während ein anderer Teil als Gleitfläche zum Zusammenwirken mit einer anderen Prothesenkomponente bestimmt ist. Für die Gleitfläche ist eine erhöhte Oberflächenrauigkeit nicht gewünscht. Bei dem erfindungsgemäßen Verfahren kann deswegen vorgesehen sein, dass nur ein Teil der Oberfläche des Keramikbauteils mit dem Strahlgut behandelt wird, während ein anderer Teil der Oberfläche von der Behandlung mit dem Strahlgut ausgespart bleibt. Wird im Rahmen der Erfindung von einer Behandlung der Oberfläche des Keramikbauteils gesprochen, so kann damit die gesamte Oberfläche oder ein Teil der Oberfläche gemeint sein.

Die Teilchengröße des Strahlguts liegt vorzugsweise in derselben Größenordnung wie die Teilchengröße des Keramikausgangsmaterials. Das Strahlgut kann dann besonders wirksam auf das Keramikbauteil einwirken. Allerdings besteht bei der Verwendung eines solchen Strahlguts die Gefahr, dass sich Partikel des Strahlguts in den Lücken des Keramikmaterials festsetzen, die durch die herausgeschlagenen Bruchstücke entstehen. Die Partikel stellen dann Verunreinigungen im Keramikmaterial dar. Verunreinigungen dieser Art können verhindert werden, indem als Strahlgut ein Pulver verwendet wird, das dem Pulver des Keramikausgangsmaterials entspricht. Setzen sich Partikel dieses Materials in dem Keramikbauteil fest, so ändern sie nichts an der homogenen Zusammensetzung des Keramikmaterials.

Die auf die Oberfläche des Keramikbauteils aufzubringende Beschichtung kann aus einer Titan-Legierung oder reinem Titan bestehen. Als Verfahren zum Aufbringen der Beschichtung kommt Plasmaspritzen in Frage. Das Plasmaspritzen ist ein Verfahren, bei dem ein Gas durch einen Lichtbogen geleitet und dadurch ionisiert wird. Das Beschichtungsmaterial wird in Pulverform in das ionisierte Gas eingebracht und von dem Gasstrom auf das zu beschichtende Werkstück transportiert.

Als geeignete Keramikwerkstoffe für das erfindungsgemäße Verfahren haben sich Zirkoniumoxid, Aluminiumoxid sowie Mischungen aus beiden erwiesen.

Die Erfindung wird nachfolgend unter Bezugnahme auf die beigefügten Zeichnung anhand einer vorteilhaften Ausführungsform beispielhaft beschrieben. Es zeigen:
- Fig. 1: eine Bandscheibenprothese in Schnittdarstellung;
- Fig. 2: einen vergrößerten Ausschnitt aus der Bandscheibenprothese gemäß Fig. 1;
- Fig. 3: eine schematische Darstellung der inneren Struktur des Keramikmaterials im Ausgangszustand;
- Fig. 4: die Ansicht aus Fig. 3 nach dem Vorsintern; und
- Fig. 5: die Ansicht aus Fig. 3 nach dem Sintern.

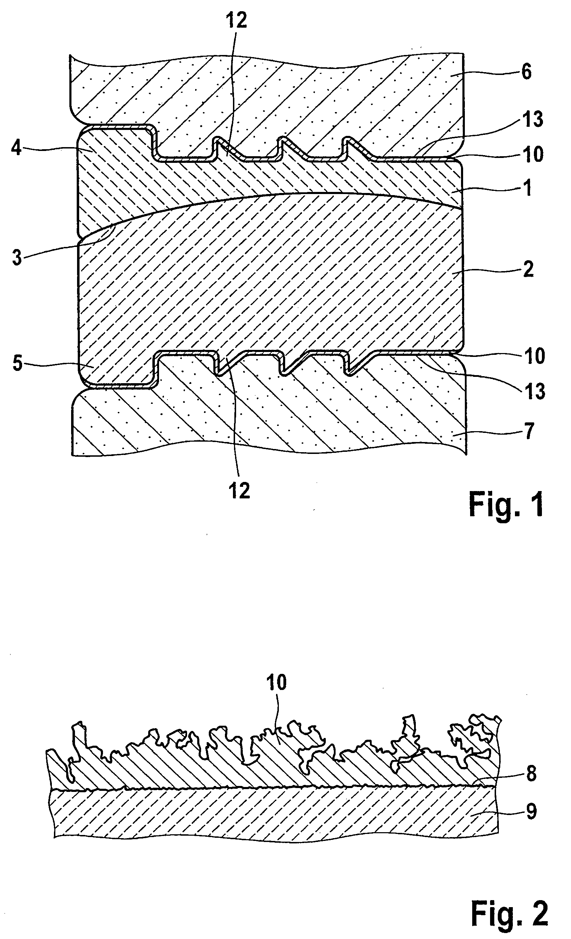

In Fig. 1 ist eine als Bandscheibenprothese ausgebildete Endoprothese in einen Zwischenwirbelraum zwischen zwei Wirbelkörpern 6, 7 eingesetzt. Die Bandscheibenprothese umfasst eine erste Anschlussplatte 1 und eine zweite Anschlussplatte 2. Die erste Anschlussplatte 1 ist eine zur Verbindung mit einem ersten Wirbelkörper 6 ausgebildete Endoprothesenkomponente, die zweite Anschlussplatte 2 ist eine zur Verbindung mit einem zweiten Wirbelkörper 7 ausgebildete Endoprothesenkomponente.

Die erste Anschlussplatte 1 und die zweite Anschlussplatte 2 liegen über zueinander passende Gleitflächen 3 aneinander an. Die Gleitflächen 3 bilden ein Gelenk für Bewegungen zwischen der oberen Anschlussplatte 1 und der unteren Anschlussplatte 2.

In Bereichen 13 der Oberflächen der Anschlussplatten 1, 2, mit denen die Anschlussplatten 1, 2 an dem Knochenmaterial der Wirbelkörper 6, 7 anliegen, sind Vorsprünge 12 ausgebildet. Die Vorsprünge 12 haben eine flachere Flanke in der Richtung, in der die Anschlussplatten 1, 2 in den Zwischenwirbelraum eingeschoben werden, und eine steilere Flanke in entgegengesetzter Richtung. Durch die flachere Flanke wird das Einschieben der Anschlussplatten 1, 2 in den Zwischenwirbelraum erleichtert. Die steilere Flanke bietet den Anschlussplatten 1, 2 Halt, wenn die Vorsprünge 12 in das Knochenmaterial der Wirbelkörper 6, 7 eingedrungen sind. Die steileren Flanken verhindern, dass die Anschlussplatten 1, 2 in entgegengesetzter Richtung wieder aus dem Zwischenwirbelraum herausgezogen werden können.

Flansche 4, 5 der Anschlussplatten 1, 2 sind dazu bestimmt, an der ventralen Seite der Wirbelkörper 6, 7 anzuliegen. Wegen der Flansche 4, 5 können die Anschlussplatten 1, 2 in dorsaler Richtung nicht über die gewünschte Position hinaus in den Zwischenwirbelraum eingeschoben werden.

Die Anschlussplatte 1 und die Anschlussplatte 2 sind aus einem Keramikwerkstoff 9 geformt. Die Gleitflächen 3 an den Anschlussplatten 1, 2 sind an einer Oberfläche des Keramikwerkstoffs 9 ausgebildet. Die Bereiche 13, über die eine Verbindung zu dem Knochenmaterial hergestellt wird, sind mit einer Beschichtung 10 aus einer Titan-Legierung belegt.

Fig. 2 zeigt einen vergrößerten Ausschnitt aus einer Anschlussplatte 1, 2, in dem die Grenzfläche 8 zwischen dem Keramikwerkstoff 9 und der Beschichtung 10 gezeigt ist. Der Keramikwerkstoff 9 hat an der Grenzfläche 8 zur Beschichtung 10 eine Rauigkeit Rₐ, die mindestens 2,5 µm beträgt. Diese Rauigkeit an der Grenzfläche 8 reicht aus, um eine stabile Verbindung mit der Beschichtung 10 einzugehen, für eine stabile Verbindung mit Knochenmaterial reicht sie nicht aus. Die Beschichtung 10 hat eine größere Rauigkeit und größere Porosität als der Keramikwerkstoff 9 und geht deswegen eine stabile Verbindung mit dem Knochenmaterial ein.

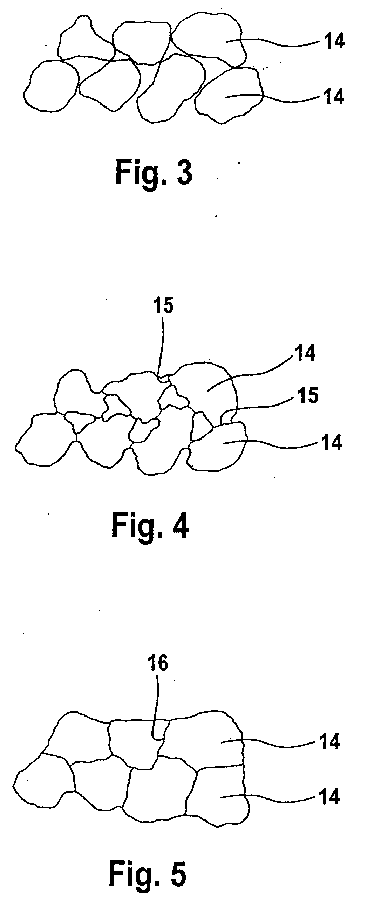

Um ein Keramikbauteil in der Gestalt einer Endoprothesenkomponente herzustellen, bei dem ein Teil der Oberfläche die gewünschte Rauigkeit hat, wird zunächst ein in Pulverform vorliegendes Ausgangsmaterial in eine Form eingebracht, deren Gestalt dem zu erzeugenden Keramikbauteil entspricht. Da das Keramikbauteil durch die nachfolgenden Verfahrensschritte an Volumen verliert, hat die Form größerer Abmessungen als das fertige Keramikbauteil. In der Form wird das Pulver mechanisch verdichtet, beispielsweise durch Schütteln und Druck, die Partikel 14 des Pulvers liegen dann nebeneinander, wie es in Fig. 3 schematisch dargestellt ist. Durch das Vorsintern bei einer Temperatur von 920°C bilden sich in Fig. 4 dargestellte Brücken zwischen den Partikeln 14. Die innere Struktur des Materials ist nun so stabil, dass das Keramikbauteil aus der Form herausgenommen werden kann.

Mit den Brücken 15 hat das Keramikmaterial eine größere Stabilität in seiner Struktur, als man sie für eine mechanische Bearbeitung durch Bohren oder Fräsen wählen würde. Die Stabilität der Struktur ist gerade so eingestellt, dass durch Bestrahlen mit einem Pulver, das dem Ausgangsmaterial entspricht, Bruchstücke aus dem vorgesinterten Keramikmaterial herausgelöst werden können. Die Bruchstücke sind so groß, dass sich nach dem Sintern bei dem fertigen Keramikbauteil die gewünschte Oberflächenrauigkeit ausbildet.

Ist die gewünschte Oberflächenstruktur erreicht und die mechanische Bearbeitung abgeschlossen, wird das Keramikbauteil bei einer Temperatur gesintert, die höher liegt als die Temperatur des Vorsinterns. An den Grenzflächen der bislang nur über die Brücken 15 verbundenen Partikel bilden sich flächige Verbindungen 16. Da die Hohlräume aus dem Inneren verschwinden, nimmt das Volumen des Keramikbauteils weiter ab. Auf dem Teil der Oberfläche, der mit dem Strahlgut behandelt wurde, hat die Oberfläche nun eine Rauigkeit Rₐ von ungefähr 4 µm. Eine auf diesen Oberflächenanteil aufgebrachte Beschichtung aus einer Titan-Legierung haftet fest auf der Oberfläche.

## Patentansprüche

1. Verfahren zum Herstellen eines Keramikbauteils mit folgenden Schritten:
a. Bereitstellen eines Keramikausgangsmaterials in Pulverform;
b. Bereitstellen einer die Gestalt des Keramikbauteils vorgebenden Form;
c. Einbringen des Keramikausgangsmaterials in die Form;
d. Vorsintern des Keramikbauteils;
e. Herauslösen des Keramikbauteils aus der Form;
g. Sintern des Keramikbauteils bei einer Temperatur, die höher ist als die Temperatur des Vorsinterns ; **dadurch gekennzeichnet, dass**:
- die Temperatur des Vorsinterns zwischen 880°C und 980°C liegt
- zwischen den Schritten e und g, ein weiterer Schritt f. Zum Behandeln der Oberfläche des Keramikbauteils mit einem Strahlgut vorgesehen ist.
;

2. Verfahren nach Anspruch 1, **dadurch gekennzeichnet, dass** die Temperatur des Vorsinterns größer ist als 900°C.

3. Verfahren nach Anspruch 1 oder 2, **dadurch gekennzeichnet, dass** die Temperatur des Vorsinterns kleiner ist als 950°C.

4. Verfahren nach einem der Ansprüche 1 bis 3, **dadurch gekennzeichnet, dass** die Rauigkeit Rₐ des Keramikbauteils auf einem Oberflächenanteil größer ist als 2,5 µm.

5. Verfahren nach einem der Ansprüche 1 bis 4, **dadurch gekennzeichnet, dass** ein Strahlgut verwendet wird, dessen Partikelgröße der Partikelgröße des Keramikausgangsmaterials entspricht.

6. Verfahren nach einem der Ansprüche 1 bis 5, **dadurch gekennzeichnet, dass** ein dem Keramikausgangsmaterial entsprechendes Material als Strahlgut verwendet wird.

7. Verfahren nach einem der Ansprüche 1 bis 6, **dadurch gekennzeichnet, dass** eine Beschichtung aus einer Titan-Legierung auf die Oberfläche des Keramikbauteils aufgebracht wird.

8. Verfahren nach einem der Ansprüche 1 bis 7, **dadurch gekennzeichnet, dass** die gesamte Oberfläche des Keramikbauteils mit dem Strahlgut behandelt wird.

9. Verfahren nach einem der Ansprüche 1 bis 7, **dadurch gekennzeichnet, dass** ein Teil der Oberfläche des Keramikbauteils von der Behandlung mit dem Strahlgut ausgespart bleibt.

## Claims

1. Method for producing a ceramic component, said method comprising the following steps:
a. making available a ceramic starting material in powder form;
b. making available a mold that predefines the shape of the ceramic component;
c. introducing the ceramic starting material into the mold;
d. presintering the ceramic component;
e. removing the ceramic component from the mold;
g. sintering the ceramic component at a temperature that is higher than the temperature of the presintering;
**characterized in that**:
- the temperature of the presintering is between 880°C and 980°C;
- between steps e and g a further step f.
is provided for treating the surface of the ceramic component with a blasting material.

2. Method according to Claim 1, **characterized in that** the temperature of the presintering is higher than 900°C.

3. Method according to Claim 1 or 2, **characterized in that** the temperature of the presintering is lower than 950°C.

4. Method according to one of Claims 1 to 3, **characterized in that** the roughness Rₐ of the ceramic component on a portion of the surface is greater than 2.5 µm.

5. Method according to one of Claims 1 to 4, **characterized in that** a blasting material is used whose particle size corresponds to the particle size of the ceramic starting material.

6. Method according to one of Claims 1 to 5, **characterized in that** a material corresponding to the ceramic starting material is used as blasting material.

7. Method according to one of Claims 1 to 6, **characterized in that** a coating composed of a titanium alloy is applied to the surface of the ceramic component.

8. Method according to one of Claims 1 to 7, **characterized in that** the entire surface of the ceramic component is treated with the blasting material.

9. Method according to one of Claims 1 to 7, **characterized in that** a part of the surface of the ceramic component remains excluded from the treatment with the blasting material.

## Revendications

1. Procédé de fabrication d'un composant céramique comprenant les étapes suivantes :
a. la mise à disposition d'un matériau de départ céramique sous forme de poudre ;
b. la mise à disposition d'un moule donnant la forme du composant céramique ;
c. l'introduction du matériau de départ céramique dans le moule ;
d. le pré-frittage du composant céramique ;
e. l'extraction du composant céramique du moule ;
g. le frittage du composant céramique à une température supérieure à la température du pré-frittage ;
**caractérisé en ce que** :
- la température du pré-frittage se situe entre 880 °C et 980 °C ;
- une étape f supplémentaire pour le traitement de la surface du composant céramique avec une matière de projection est prévue entre les étapes e et g.

2. Procédé selon la revendication 1, **caractérisé en ce que** la température du pré-frittage est supérieure à 900 °C.

3. Procédé selon la revendication 1 ou 2, **caractérisé en ce que** la température du pré-frittage est inférieure à 950 °C.

4. Procédé selon l'une quelconque des revendications 1 à 3, **caractérisé en ce que** la rugosité Rₐ du composant céramique sur une proportion de la surface est supérieure à 2,5 µm.

5. Procédé selon l'une quelconque des revendications 1 à 4, **caractérisé en ce qu'**une matière de projection dont la taille de particule correspond à la taille de particule du matériau de départ céramique est utilisée.

6. Procédé selon l'une quelconque des revendications 1 à 5, **caractérisé en ce qu'**un matériau correspondant au matériau de départ céramique est utilisé en tant que matière de projection.

7. Procédé selon l'une quelconque des revendications 1 à 6, **caractérisé en ce qu'**un revêtement en un alliage de titane est appliqué sur la surface du composant céramique.

8. Procédé selon l'une quelconque des revendications 1 à 7, **caractérisé en ce que** l'ensemble de la surface du composant céramique est traitée avec la matière de projection.

9. Procédé selon l'une quelconque des revendications 1 à 7, **caractérisé en ce qu'**une partie de la surface du composant céramique reste épargnée par le traitement avec la matière de projection.
